Europäisches Patentamt

European Patent Office

Office européen des brevets

(19)

(11) EP 1 231 468 A1

(12) EUROPEAN PATENT APPLICATION
published in accordance with Art. 158(3) EPC

(43) Date of publication:
14.08.2002 Bulletin 2002/33

(51) Int Cl.7: G01N 33/53, G01N 33/543,
G01N 33/566

(21) Application number: 00911341.6

(22) Date of filing: 24.03.2000

(86) International application number:
PCT/JP00/01813

(87) International publication number:
WO 01/27619 (19.04.2001 Gazette 2001/16)

(84) Designated Contracting States:
AT BE CH CY DE DK ES FI FR GB GR IE IT LI LU
MC NL PT SE

(30) Priority: 14.10.1999 JP 29303799

(71) Applicants:
• Central Research Institute of Electric Power
Industry
Tokyo 100-0004 (JP)
• Sapidyne Instruments Inc.
Boise, ID 83706-6700 (US)

(72) Inventors:
• OMURA, Naoya
Centr.Res.Inst.of Electric Power Ind.
Abiko-shi Chiba 270-1166 (JP)
• LACKIE, Steve Sapidyne Instrument Inc.
Boise, ID 83706-6700 (US)

(74) Representative: Dealtry, Brian
Eric Potter Clarkson,
Park View House,
58 The Ropewalk
Nottingham NG1 5DD (GB)

(54) METHOD FOR DETECTING EXOGENOUS ENDOCRINE DISRUPTING CHEMICAL AND DETECTION APPARATUS

(57) A method and an apparatus for conveniently detecting at a high sensitivity an extrinsic endocrine disrupter which is a so-called environmental hormone, and enabling detection at a high sensitivity of an extrinsic endocrine disrupter (environmental hormone) existing in the environment with a low concentration to be conveniently carried out. The present invention includes: a detection container (1) having an immobilized female sex hormone receptor (2) such as an immobilized estrogen receptor filled therein; a light source (3) for exciting the inside of the container; and a detector (4) detecting a fluorescence intensity in the detection container after excitation. A fluorescence intensity $F_0$ detected when a fluorescent-labeled female sex hormone having a certain concentration is added into the detection container is compared with a fluorescence intensity $F_1$ detected when a fluorescent-labeled female sex hormone having the same concentration and being mixed with a sample to be tested is added into the detection container, and it is determined that an environmental hormone exists in the sample if $F_1 < F_0$ is established. Further, the female sex hormone can be also detected by the antigen-antibody reaction utilizing an anti-hormone antibody relative to the female sex hormone.

Fig. 1

**Description**

TECHINICAL FIELD

The present invention relates to a method and an apparatus for detecting an extrinsic endocrine disrupter, and more particularly to a method and an apparatus for conveniently detecting at a high sensitivity an extrinsic endocrine disrupter which is so called an environmental hormone.

BACKGROUND ART

In recent years, extrinsic endocrine disrupters which have become a social problem, namely, so-called environmental hormones are considered to adversely affect organisms by disrupting the action of natural hormones in bodies. Although the disrupting action is currently yet to be fully clarified, a disrupting mechanism of a specific type of female sex hormone action is proposed. The female sex hormone action is usually induced when the female sex hormone is bonded to a corresponding hormone receptor in a body. A conjugate of the hormone and its receptor becomes dimeric, and is further bonded to a specific sequence of DNA. As a result, transcription of genes required for the hormone action starts, which results in the physiological function. Here, the extrinsic endocrine disrupter disrupts the above-described hormone action when bonded to the receptor in place of the natural female sex hormone.

Such extrinsic endocrine disrupters must be prevented from being diffused into the environment and eliminated from the environment, but for these, existence of the disrupting chemicals must be first detected. However, since the disrupting chemicals which exist in the environment with the very thin concentration, i.e., the unit of nanomole or picomole and whose malign influence upon organisms is worried must be detected, a special method with the best possible sensitivity must be adopted for the detection.

As prior art methods for detecting extrinsic endocrine disrupters, there are known a radioreceptor assay [Endocrine, 138(3);868-870(1997)], an immunoassay ["Reproductive Toxicology", del Mazo eds., Plenum Press, New York (1998)], a fluorescence depolarization [Environmental Health Respectives., 106(9);551-557(1998)], as well s a method using a recombinant yeast or a method using a bond of a complex with a receptor and a gene as an index by utilizing radioactivity.

However, as to these conventional methods, the operation is complicated since a radioactive material or a recombinant yeast is used, and the sensitivity of all of these methods is not sufficient for detecting the extrinsic endocrine disrupter in the environment. Therefore, development of a system which conveniently detects the extrinsic endocrine disrupter at a high sensitivity is demanded.

DISCLOSURE OF INVENTION

It is an object of the present invention to provide a novel method for detecting an extrinsic endocrine disrupter. It is another object of the present invention to provide a method and an apparatus capable of conveniently detecting at a high sensitivity an extrinsic an endocrine disrupter which exists in the environment with a very low concentration and is a so-called environmental hormone.

To achieve this aim, as a result of eagerly carrying on studies, the present inventor has turned the attention to the fact that most of chemical substances suspicious as environmental hormones have the action similar to female sex hormones and has found that existence of the environmental hormones in a sample can be conveniently detected at a high sensitivity by combining a female sex hormone receptor with a fluorescent-labeled female sex hormone and detecting a result of competitive bonding of the female sex hormone and the environmental hormone to the receptor by using a difference in intensity of fluorescence, thereby completing the present invention.

That is, according to the present invention achieving the above objects, there is provided a method for detecting existence of an extrinsic endocrine disrupter in a sample, wherein an immobilized female sex hormone receptor and a fluorescent-labeled female sex hormone are used, there is performed the competitive bonding reaction between an already-known quantity of the fluorescent-labeled female sex hormone and an extrinsic endocrine disrupter whose existence in the sample is unknown to an already-known quantity of the immobilized female sex hormone receptor, and the fluorescence intensity of the fluorescent label of the fluorescent-labeled female sex hormone bonded to the immobilized female sex hormone receptor is measured.

Furthermore, according to a first embodiment of the method for detecting an extrinsic endocrine disrupter of the present invention, assuming that $F_0$ is a detection value of the fluorescence intensity detected when a fluorescent-labeled female sex hormone with a certain concentration is added into a detection container having an immobilized female sex hormone receptor filled therein and $F_1$ is a detection value of the fluorescence intensity detected when a fluorescent-labeled female sex hormone having the same concentration as the above and being mixed with a sample to be tested is added into the detection container, when the detection value $F_1$ is smaller than the detection value $F_0$,

it is determined that an extrinsic endocrine disrupter exists in the sample. As a result, the qualitative detection can be readily performed.

According to a second embodiment of the method for detecting an extrinsic endocrine disrupter of the present invention, assuming that $F_0$ is a detection value of the fluorescence intensity detected when a fluorescent-labeled female sex hormone with a certain concentration is added into a detection container having an immobilized female sex hormone receptor filled therein and $F_1$ is a detection value of the fluorescence intensity detected when a fluorescent-labeled female sex hormone having the same concentration as the above and being mixed with a sample is added into the detection container, a ratio of the detection value $F_1$ and the detection value $F_0$ is calculated, and a quantity of an extrinsic endocrine disrupter existing in the sample is obtained by a previously created calibration curve. Consequently, the quantitative detection is enabled.

Moreover, the present invention provides the method for detecting an extrinsic endocrine disrupter, wherein the female sex hormone receptor is an estrogen receptor. Many chemicals having the female sex hormone-like action which exist in the environment can be detected by using the estrogen receptor.

In addition, the present invention provides the method for detecting an extrinsic endocrine disrupter, wherein the detection container is a cell having a cubic capacity of not more than 100 mm$^3$. By using such a cell, detection can be further rapidly carried out with a small quantity of the immobilized female sex hormone receptor.

Additionally, the present invention provides the method for detecting an extrinsic endocrine disrupter, wherein the detection container is of a flow type so that the sample can be continuously brought into contact with the immobilized female sex hormone receptor. When the detection container is of a flow type, a disrupting chemical is accumulated in the immobilized female sex hormone receptor in the detection container even if only a very small quantity of the extrinsic endocrine disrupter exists in the sample, thereby further improving the limit in detection.

According to the present invention achieving the above objects, there is provided an apparatus for detecting an extrinsic endocrine disrupter, comprising: a detection container having an immobilized female sex hormone receptor filled therein; a light source which excites the inside of the detection container; and a detector which detects the fluorescence intensity in the detection container after excitation, used in combination with fluorescent-labeled female sex hormone.

Further, the present invention provides the apparatus for detecting an extrinsic endocrine disrupter, wherein the female sex hormone receptor is an estrogen receptor.

Furthermore, the present invention provides the apparatus for detecting an extrinsic endocrine disrupter, wherein the detection container is a cell whose cubic capacity is not more than 100 mm$^3$.

Moreover, the present invention provides the apparatus for detecting an extrinsic endocrine disrupter, wherein the detection container is of a flow type so that the sample can be continuously brought into contact with the immobilized female sex hormone receptor.

Based on the point to which the attention has been directed and which is similar to the method of using the female sex hormone receptor, the present inventor has found, as a result of eagerly carrying on studies, that existence of an environmental hormone in a sample can be conveniently detected at a high sensitivity by bonding an immobilized female sex hormone with an anti-hormone antibody relative to the female sex hormone, leading the anti-hormone antibody into a sample to be tested in order to cause the antigen-antibody reaction of an environmental hormone and the anti-hormone antibody, thereafter bringing a mixture obtained from this reaction into contact with the immobilized female sex hormone to bond the unreacting anti-hormone antibody existing in the mixture with the immobilized female sex hormone, and detecting a result by using a difference in the fluorescence intensity of the fluorescent label bonded to the anti-hormone antibody. The present inventor has provided further insights and completed the present invention.

That is, according to a second invention which achieves the above-described objects, there is provided a method for detecting existence of an extrinsic endocrine disrupter in a sample, comprising the steps of: using an immobilized female sex hormone and an anti-hormone antibody with respect to the female sex hormone to lead an already-known quantity of the anti-hormone antibody into the sample and subjecting it to the antigen-antibody reaction with an extrinsic endocrine disrupter whose existence is unknown; bringing a liquid mixture obtained from the reaction between anti-hormone antibody and the sample into contact with an already-known quantity of the immobilized female sex hormone; and measuring a fluorescence intensity of the fluorescent label bonded to the anti-hormone antibody, thereby detecting a quantity of the anti-hormone antibody bonded to the immobilized female sex hormone. As described above, when detecting the environmental hormone by using the antibody, all environmental hormones to which the antibody can be bound can be specified, and the concentration of such hormones can be quantitatively detected.

The method for detecting an extrinsic endocrine disrupter according to the second invention is characterized in that, according to a first embodiment, the anti-hormone antibody is a fluorescent-labeled anti-hormone antibody, and a quantity of the anti-hormone antibody bonded to the immobilized female sex hormone is detected by measuring a fluorescence intensity of the fluorescent label of the fluorescent-labeled anti-hormone antibody.

The method for detecting an extrinsic endocrine disrupter according to the second invention is characterized in that, according to a second embodiment, the anti-hormone antibody is determined as a non-labeled primary antibody, and a quantity of the anti-hormone antibody bonded to the immobilized female sex hormone is detected by using the fluorescent-labeled anti-primary-antibody antibody specifically bonded to the primary antibody as a secondary antibody, specifically bonding the secondary antibody to the primary antibody bonded to the immobilized female sex hormone and measuring a fluorescence intensity of the fluorescent label of the bonded secondary antibody.

The method for detecting an extrinsic endocrine disrupter according to the second invention is characterized in that, in another embodiment, assuming that $F_0$ is a detection value of a fluorescence intensity detected when the anti-hormone antibody with a certain concentration is added into a detection container having the immobilized female sex hormone filled therein and $F_1$ is a detection value of a fluorescence intensity detected when the anti-hormone antibody having the same concentration as the above and being mixed with a sample to be tested is added into the detection container, when the detection value $F_1$ is smaller than the detection value $F_0$, it is determined that an extrinsic endocrine disrupter exists in the sample.

The method for detecting an extrinsic endocrine disrupter according to the second invention is characterized in that, in still another embodiment, assuming that $F_0$ is a detection value of a fluorescence intensity detected when the anti-hormone antibody with a certain concentration is added into a detection container having the immobilized female sex hormone filled therein and $F_1$ is a detection value of a fluorescence intensity detected when the anti-hormone antibody with the same concentration as the above and being mixed with a sample to be tested is added into the detection container, a ratio of the detection value $F_1$ and the detection value $F_0$ is calculated and a quantity of the extrinsic endocrine disrupter existing in the sample is obtained by using a previously created calibration curve.

Further, the present invention provides the method for detecting an extrinsic endocrine disrupter, wherein the detection container is a cell whose cubic capacity is not more than 100 mm$^3$. By using such a cell, a difference in fluorescence intensity between small quantities of samples can be detected.

Furthermore, the present invention provides the method for detecting an extrinsic endocrine disrupter, wherein the detection container is of a flow type so that the sample can be continuously brought into contact with the immobilized female sex hormone receptor.

Moreover, the above-described objects can be achieved by an apparatus for detecting an extrinsic endocrine disrupter, comprising: a detection container having an immobilized female sex hormone filled therein; a light source which excites the inside of the detection container; and a detector which detects a fluorescence intensity inside the detection container after excitation, the apparatus is used in combination with an anti-hormone antibody relative to the fluorescent-labeled female sex hormone.

In addition, the above-described objects can be attained by an apparatus for detecting an extrinsic endocrine disrupter, comprising: a detection container having an immobilized female sex hormone filled therein; a light source which excites the inside of the detection container; and a detector which detects a fluorescence intensity inside the detection container after excitation, the apparatus is used in combination with an anti-hormone primary antibody relative to the female sex hormone and a fluorescent-labeled secondary antibody specifically bonded to the anti-hormone antibody.

BRIEF DESCRIPTION OF DRAWINGS

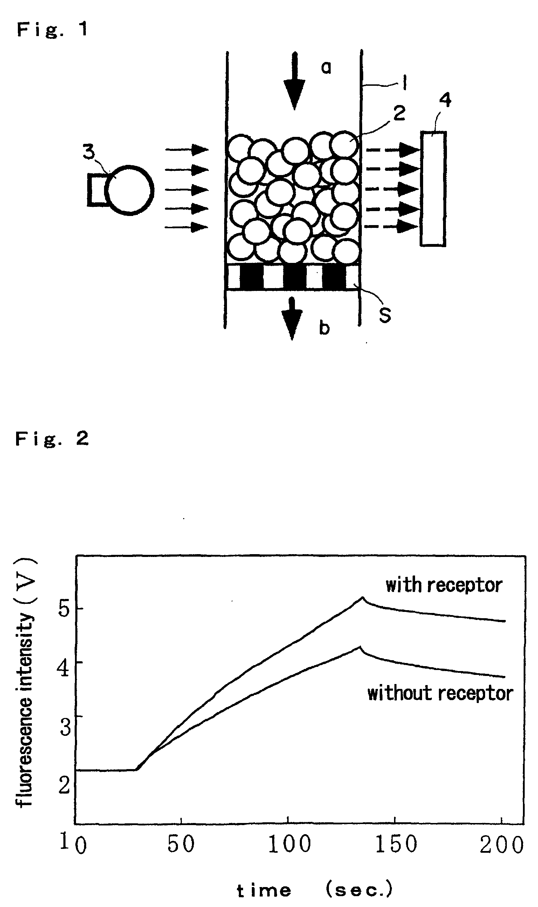

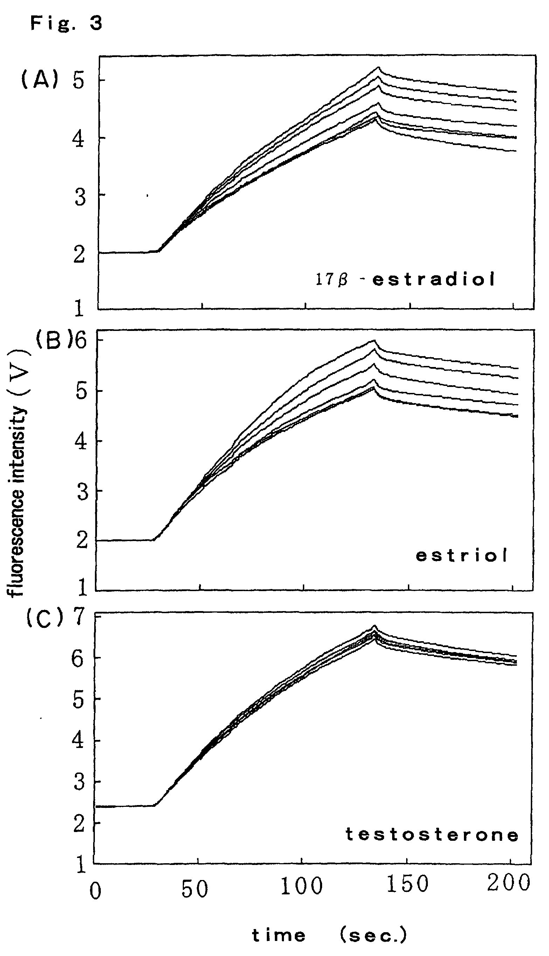

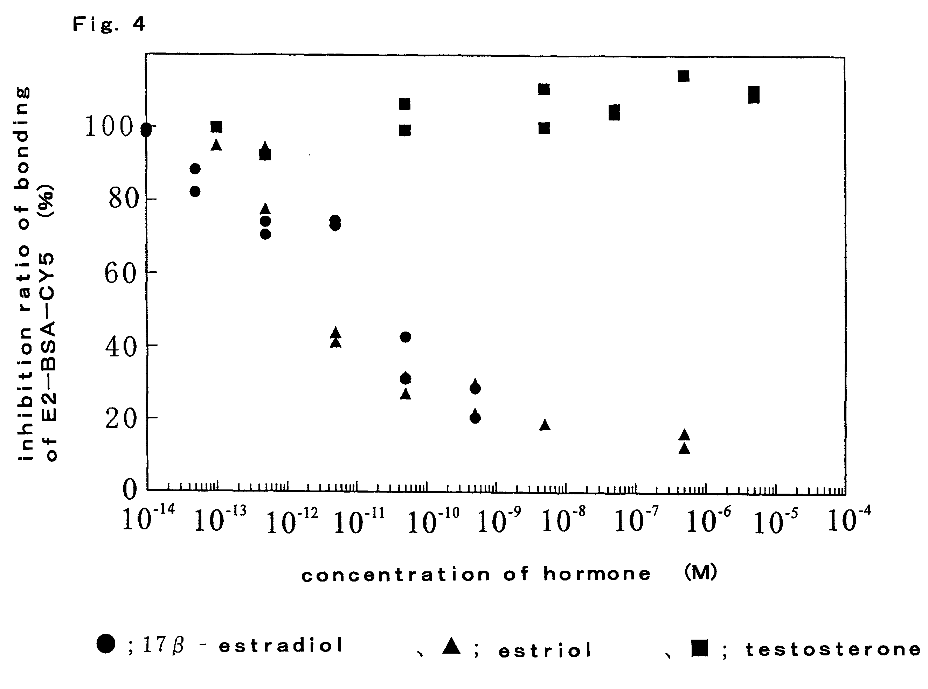

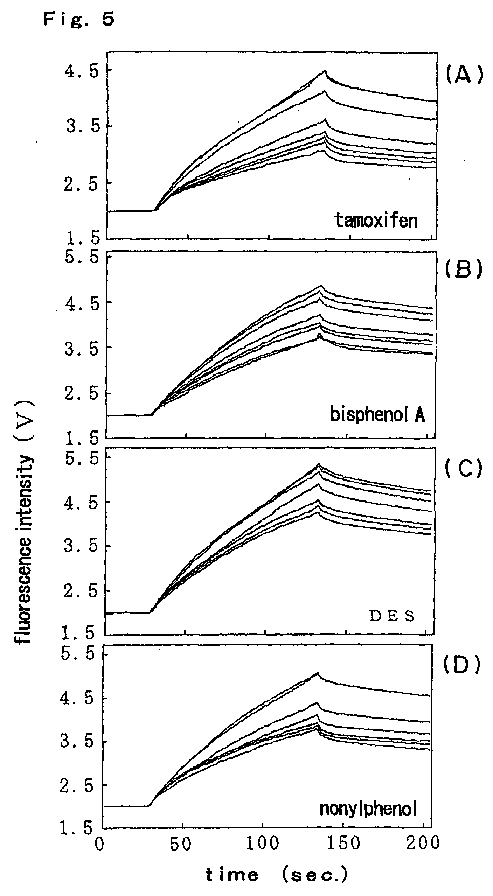

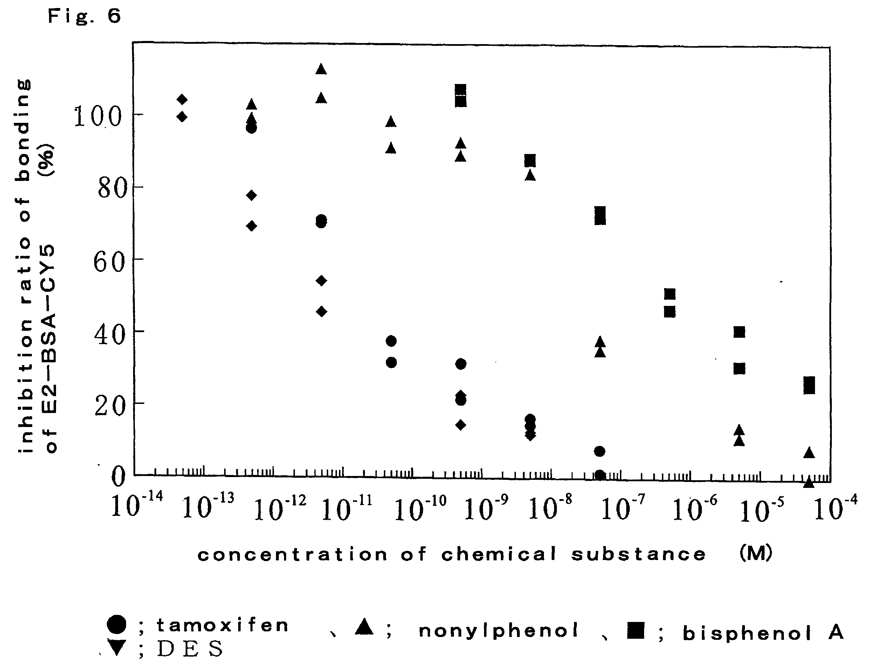

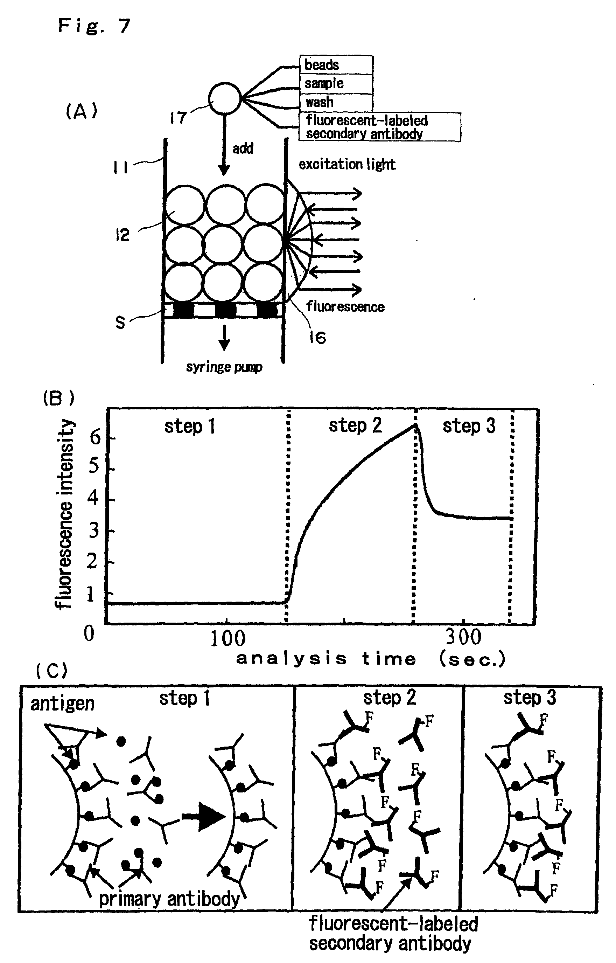

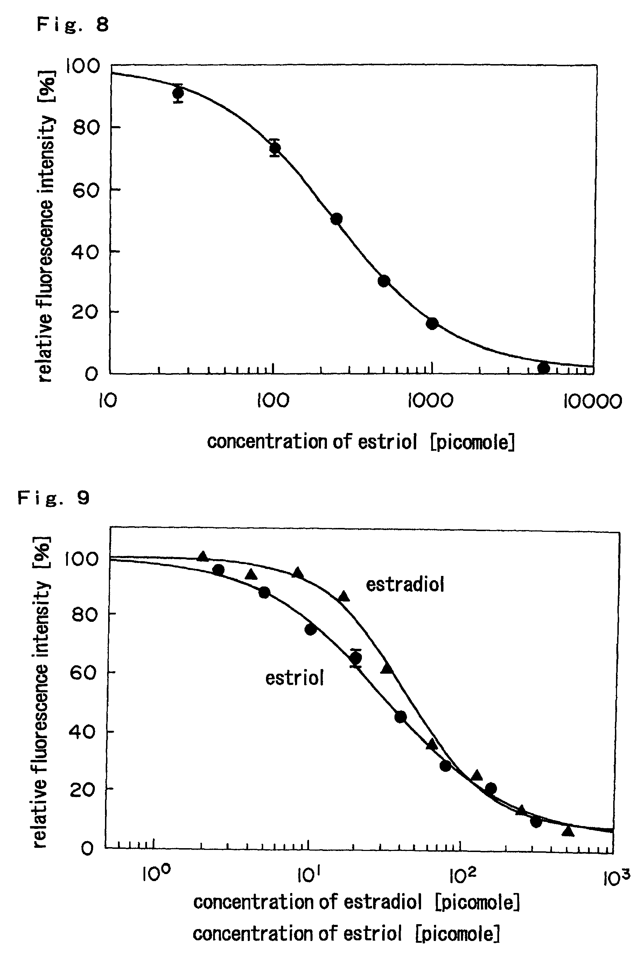

Fig. 1 is a view typically showing an apparatus for detecting an extrinsic endocrine disrupter according to the present invention; Fig. 2 is a graph showing detection of fluorescence accumulation when a female sex hormone sample is bonded to an estrogen receptor; Fig. 3 is a graph showing changes in fluorescence intensity due to coexistence of a natural hormone, wherein a natural hormone (A) is 17 β -estradiol, (B) is estriol and (C) is testosterone; Fig. 4 is a graph showing changes in inhibition ratio of bonding due to concentrations of various kinds of hormones; FIG. 5 is a graph showing changes in fluorescence intensity due to coexistence of chemical substances, wherein a chemical substance (A) is tamoxifen, (B) is bisphenol A, (C) is DES and (D) is nonylphenol; Fig. 6 is a graph showing changes in inhibition ratio of bonding due to concentrations of various kinds of chemical substances; Figs. 7 are views typically showing another method and apparatus for detecting an extrinsic endocrine disrupter according to the present invention, wherein (A) shows an example of a structure of an apparatus to be used, (B) shows a typical example of changes in fluorescence intensity detected in a series of steps of a detection operation and (C) is a type drawing showing changes in the vicinity of a surface of an antigen-immobilized bead in a series of steps of a detection operation; Fig. 8 is a graph showing a result of quantative determination of estriol measured in a system in which an anti-hormone antibody is fluorescent-labeled; and Fig. 9 is a graph showing a result of quantative determination of estriol and estradiol measured in a system using a fluorescent-labeled secondary antibody.

BEST MODE FOR CARRYING OUT THE INVENTION

The present invention will now be described in detail hereinafter based on a preferred embodiment.

It is to be noted that an immobilized female sex hormone receptor means a substance obtained by immobilizing a female sex hormone receptor in an insoluble carrier.

As a female sex hormone receptor, for example, an estrogen receptor, a gestagen receptor and others can be used. The estrogen receptor is preferable since various kinds of extrinsic endocrine disrupters can be bound thereto. Here, the estrogen receptor is also called a follicular hormone receptor, and it exists in a target cell nucleus of estrogen in an organism and mediates activation of protein synthesis which depends on a specific gene when bonded to estrogen.

On the other hand, as an insoluble carrier for immobilizing such a female sex hormone receptor, for example, polymethyl methacrylic acid, beads for immobilization consisting of, e.g., glass, fine particles such as calcium alginate particles can be used. However, the present invention is not restricted thereto, and materials having various kinds of shapes and qualities can be used. Among these materials, one having a shape of beads or fine particles, or that having a shape of beads or fine particles with an average particle size of 50 to 100 μm in particular is preferable.

As a method for immobilizing the female sex hormone receptor with respect to such an insoluble carrier, the present invention is not restricted to a specific method. For example, bonding can be carried out by using a natural adsorption, an ionic bond, a covalent bond and others. Further, not only the method of direct bonding can be used, but also indirect bonding through a spacer such as an appropriate chemical substance or protein is possible.

In the present invention, any kind of the female sex hormone used for obtaining a reference of a fluorescence intensity can be adopted as long as it has an estrus action. As examples of such a female sex hormone, there are a steroid hormone such as estradiol, estrone, estriol, equilin, erkinin, or its metabolite, chemical derivatives of these substances such as homoestrone, ethynyl estradiol, doisynolic acid, or synthetic estrogen. Fluorescent labeling of these female sex hormones can be carried out by conventional means. As a fluorescence dye for fluorescent labeling, there are CY5, fluorescein isothiocyanate, tetramethyl rhodamine isothiocyanate and others.

When an extrinsic endocrine disrupter exists in various kinds of samples to be tested in the environment where existence of the disrupter should be tested, the present invention detects the disrupter by utilizing a phenomenon that this disrupter is bonded to the female sex hormone receptor. Thus, a fluorescence intensity is first measured when the fluorescent-labeled female sex hormone with the specific concentration is bonded to the female sex hormone receptor (this value is determined as a detection value $F_0$). A fluorescence intensity is then measured when the fluorescent-labeled female sex hormone having the same concentration as the above to which a sample to be tested is mixed is bonded to the female sex hormone receptor (this value is determined as a detection value $F_1$). Here, if the extrinsic endocrine disrupter exists in the sample, bonding of the female sex hormone to the estrogen receptor is inhibited, and hence its result is represented as a drop in the fluorescence intensity in the detection value $F_1$. Therefore, when the detection value $F_1$ is smaller than the detection value $F_0$ ($F_1 < F_0$), it can be determined that the extrinsic endocrine disrupter exists in the sample.

As described above, in the present invention, a detection container having an immobilized female sex hormone held therein is used for measuring the fluorescent label of the fluorescent-labeled female sex hormone bonded to the immobilized female sex hormone receptor, which is preferable for carrying out rapid measurement.

The detection container must hold the immobilized female sex hormone receptor therein and not inhibit detection of the fluorescence from the fluorescent-labeled female sex hormone bonded to the receptor. For example, the detection container consists of transparent glass, transparent plastic or the like. The detection container may have a shape such that the bottom is closed so that a sample to be led does not flow (in this case, each measurement of the fluorescence intensity can be performed in the batch style). However, when the detection container has a conformation that the immobilized receptor is held and restrained at a predetermined position in the open container so as to enable transmission of a liquid by a technique for setting at the opened bottom a holding material such as a screen which does not pass the immobilized receptor but pass the introduced sample therethrough (when the detection container is of a so-called flow type), the sample can be continuously added into the detection container. As a result, even in case of a sample which includes a very small amount of a target substance to be bonded to the receptor, the target substance can be accumulated/concentrated in the detection container and the detection limit concentration of the target substance is consequently improved, which is preferable. Incidentally, as to a method for holding the immobilized receptor in the flow type detection container, the present invention is not restricted to the above-described method using a holding material such as a screen. Besides this method, it is possible to adopt a method in which an aggregated material having a dipping property is used as a carrier for immobilizing the receptor and it is arranged so as to close the cross section of the flow path of the detection container.

Furthermore, it is preferable for the detection container to have a shape of a small cell so as to detect a small difference in fluorescence intensity between samples with small quantities. It is desirable that the small cell has a cubic capacity of, e.g., not more than 100 mm$^3$, and 20 to 70 mm$^3$ in particular.

The fluorescence intensity is detected by a detector which detects the fluorescence generated when exciting the fluorescent label on the female sex hormone captured to the receptor by a laser beam from an appropriate light source, irradiation of a mercury lamp or the like.

Since the present invention directly utilizes the bonding capability of the extrinsic endocrine disrupter to the female sex hormone receptor, the detection sensitivity is greatly improved as compared with the immunoassay method or the like which indirectly measures an antibody of the receptor which has not been bonded to the disrupter. Moreover, since detection is conducted by measuring the fluorescence intensity, the operation is much easier than the prior art methods which utilize radiation or a recombinant yeast. According to the present invention, therefore, it is possible to easily detect at a high sensitivity an extrinsic endocrine disrupter, namely, a so-called environmental hormone, which is known to be bonded to the hormone receptor in place of a natural female sex hormone and disrupts the hormone action in a body.

The present invention mainly carries out a so-called qualitative test that a fluorescence intensity $F_0$ in case of only the fluorescent-labeled female sex hormone is compared with a fluorescence intensity $F_1$ in case of a mixture of a sample to be tested and the female sex hormone. If $F_1$ is smaller than $F_0$, i.e., a difference represented by the expression 1 is positive, it is determined that the extrinsic endocrine disrupter exists in the sample.

$$F_0 - F_1 \qquad\qquad\qquad (1)$$

And, since the percentage of reduction in $F_1$ relative to $F_0$ is in proportion to a quantity of the extrinsic endocrine disrupter included in the sample to some extent, quantification of the extrinsic endocrine disrupter included in the sample can be carried out in accordance with the percentage of reduction in $F_1$ by previously creating a calibration curve by using the extrinsic endocrine disrupter with the already-known concentration.

The second invention will now be described.

In the second invention, as different from the first invention mentioned above, the female sex hormone is immobilized to an insoluble carrier. As the insoluble carrier and the female sex hormone to be used, there are substances similar to those described above. A method for immobilizing the female sex hormone to the carrier is not restricted to a specific type as long as it does not deactivate the hormone. For example, the hormone can be directly bonded to the carrier by using a natural adsorption, an ionic bond, a covalent bond or the like. In addition, not only the method for carrying out direct bonding can be used, but also indirect bonding through a spacer such as an appropriate chemical substance or protein can be effected. For example, it is desirable to perform bonding through covalent bonding by leading a functional group to a part which rarely have an influence on activation of the hormone, or arranging a long-chain spacer. Specifically, for example, there are a method of diazotizing a carrier having an aromatic amino group and diazo-coupling a chemical substance which can be an antigen of the hormone or the like, or a method of performing covalent bonding of an amino group of a chemical substance such as a hormone having an amino group with polysaccharide such as Sepharose by activating this polysaccharide with BrCN. Additionally, the hormone can be bonded to protein by chemical bonding, and this bonded substance can be further immobilized to the carrier. In regard to this immobilization method, the bonded substance of the hormone to protein can be directly bonded to the carrier by using the natural adsorption process, the ionic bond method, the covalent bond procedure or the like. Further, not only the direct immobilization method can be used, but also immobilization through an appropriate chemical substance, e.g., a spacer such as glutaraldehyde cross-link is possible.

Further, a fluorescent-labeled anti-hormone antibody against the female sex hormone used is additionally prepared. As the antibody, it may belong to any class of immunoglobulin which is originated from various kinds of animals. In general, a commercially available antibody can be used, or it is possible to use an antibody with the high specificity relative to the female sex hormone, which is additionally prepared by stimulating an appropriate immune system with the female hormone to be produced. Furthermore, a monoclonal antibody can be used. For example, an anti-estradiol antibody is available from companies such as Bio Design, Bio Specific, Fitzard and Coatex Bio Chemistry in the United States of America. And, an anti-estriol antibody is available from Bio Stride and others. Fluorescent labeling of such an antibody can be effected by conventional means. Furthermore, as a fluorescence dye to be used, there are materials similar to those mentioned above.

Alternatively, the anti-hormone antibody against the female sex hormone is not fluorescent-labeled and it is used as a primary antibody. Also, an antibody (anti-primary-antibody antibody) which is specifically bonded to the primary antibody can be additionally prepared, and this antibody can be fluorescent-labeled to be used as a secondary antibody. In this case, since the fluorescent-labeled secondary antibody is specifically bonded to the anti-hormone antibody (primary antibody) bonded to the immobilized female sex hormone, the anti-hormone antibody bonded to the immobilized female sex hormone can be identified by a fluorescent label as similar to the above-described conformation using the fluorescent-labeled anti-hormone antibody.

When an extrinsic endocrine disrupter exists in various kinds of samples in the environment where existence of such a disrupter should be tested, namely, the sample to be tested, the second invention detects the disrupter by utilizing a phenomenon that the disrupter is bonded to the anti-hormone antibody against the female sex hormone. Therefore, a fluorescence intensity in case of bonding the anti-hormone antibody with the certain concentration to the immobilized female sex hormone is measured (this value is determined as a detection value $F_0$). Subsequently, the

sample to be tested is brought into contact with the anti-hormone antibody with the same concentration as the above, and thereafter the mixture obtained from this contact is further brought into contact with the immobilized female sex hormone. Then, the fluorescence intensity of the anti-hormone antibody bonded to the immobilized female sex hormone is measured (this value is determined as a detection value $F_1$). Here, if the extrinsic endocrine disrupter exists in the sample, a part of the anti-hormone antibody is bonded to the extrinsic endocrine disrupter in the first contact with the sample and, in the subsequent contact with the immobilized female sex hormone, the anti-hormone antibody bonded to the extrinsic endocrine disrupter no longer tries to be bonded to the immobilized female sex hormone but remains in a mixture obtained from the reaction in the free state. Therefore, a result of this state is represented as a drop in fluorescence intensity in the detection value $F_1$. Therefore, if the detection value $F_1$ is smaller than the detection value $F_0$ ($F_1 < F_0$), it can be determined that the extrinsic endocrine disrupter exists in the sample.

Incidentally, in the second invention, it is also preferable to use the detection container having the immobilized female sex hormone held therein in order to conduct rapid measurement as similar to the first invention. In regard to the structure and the like of the detection container, the structure described in connection with the first invention can be preferably exemplified except a difference that the material held therein is the immobilized female sex hormone or the immobilized female sex hormone receptor. Moreover, the method for detecting the fluorescence is similar to that mentioned above.

The second invention is a method for indirectly measuring the extrinsic endocrine disrupter by calibrating a quantity of the antibody which has not been bonded to the extrinsic endocrine disrupter. However, since the antibody which is yet to be bonded can be accumulated to the immobilized carrier by bonding it to the antigen (female sex hormone) which exists with the high density when immobilized to the carrier, the detection sensitivity can be greatly improved and detection is carried out by measurement of the fluorescence intensity. Therefore, the operation is extremely easy as compared with the conventional methods utilizing the radiation or the recombinant yeast. Thus, according to the present invention, it is possible to easily detect at a high sensitivity the extrinsic endocrine disrupter, which is a so-called environmental hormone, known to disrupt the hormone action in a body when bonded to the hormone receptor in place of the natural female sex hormone.

Although the second invention mainly conducts the qualitative test as similar to the first invention, quantitative determination of the extrinsic endocrine disrupter included in the sample can be performed by previously creating a working curve by using the extrinsic endocrine disrupter with the known concentration as described above.

The above-mentioned method and apparatus for detecting the extrinsic endocrine disrupter according to the first and second inventions can effect detection and quantitative determination of a material having the associativity with respect to a receptor or an antibody irrespective of types of receptors or antibodies in principle. A detectable extrinsic endocrine disrupter is not restricted to a specific type, and various kinds of materials can be included. For example, there are chemical substances such as tamoxifen, nonylphenol, bisphenol A, diethylstilbestrol, estradiol-3-sulfate, estradiol-17-glucuronide, estradiol-3-glucuronide, estriol-3-sulfate, estriol-17-glucuronide, estriol-3-glucuronide and others. However, the present invention is not restricted to these several kinds of chemical substances.

EMBODIMENTS

Although preferred embodiments according to the present invention will now be described with reference to the accompanying drawings, these embodiments will be disclosed for the purpose of only helping specific and easy understanding of the present invention, and the present invention is not restricted by these embodiments.

Experiment I

A. Materials and Method

(1) Receptor, Hormone and Chemical Substances

As an estrogen receptor, a human estrogen receptor α [manufactured by PanVela Corporation, Drive Madison, Wisconsin, USA] was used. As standard brands of natural hormones, 17 β -estradiol (manufactured by Wako Pure Chemical Industries, Ltd. in Japan), estriol (manufactured by Wako Pure Chemical Industries, Ltd.) and testosterone (manufactured by Wako Pure Chemical Industries, Ltd) were used. In addition, as measurement targets, there were employed several kinds of chemical substances which are suggested to have the possibility that they have the hormone actions: tamoxifen, nonylphenol, bisphenol A, diethylstilbestrol, estradiol-3-sulfate, estradiol-17-glucuronide, estradiol-3-glucuronide, estriol-3-sulfate, estriol-17-glucuronide, estriol-3-glucuronide [all of them are manufactured by Sigma, Missouri, USA].

(2) Preparation of Immobilized Estrogen Receptor

Immobilization of the estrogen receptor to a carrier (beads) was carried out by natural adsorption. As immobilization beads, particles of polymethyl methacrylic acid having an average particle size of 95 μm (manufactured by Ganz Chemical Co., Ltd) were used. 0.2 g of the beads was put in a small test tube, and suspended in 1 ml of a buffer for bonding (50 mM tris HCl, 500 mM KCl, 2mM DDT, 1mM EDTA, 10% glycerol, pH 7.8). 70 pmol of the estrogen receptor was put in this suspension, and blended for 30 minutes at a room temperature. Thereafter, 100 μl of bovine serum albumin was added so as to obtain the final concentration of 1 mg/ml. After addition, it is further blended for 30 minutes at a room temperature. Subsequently, the particles were washed by using the same buffer, again suspended in 30 ml of the same buffer, and used for measurement. It is to be noted that, as beads for comparison in which the estrogen receptor is not immobilized, those obtained by adding only bovine serum albumin to the beads were prepared by the similar operation.

(3) Preparation of Fluorescent-labeled Female Sex Hormone

A powder sample of 17 β -estradiol-bovine serum albumin [manufactured by Sigma (mentioned above)] is dissolved in the buffer for bonding so as to obtain 1 mg/ml. As a fluorescence dye, CY5 having an absorption wavelength in 650 nm was used taking an absorption wavelength of steroid hormone and the like into consideration. In order to provide this fluorescence dye, a CY5 labeling kit [manufactured by Amersham Life Science, Arlington Heights, Illinois, USA] was used. The 17β-estradiol-bovine serum albumin having the fluorescence dye given thereto and the free fluorescence dye were partitioned by the gel filtration chromatography. As a result, there was obtained the fluorescent-labeled female sex hormone having approximately 10 fluorescence dyes given thereto with respect to one molecule of the 17 β -estradiol-bovine serum albumin.

(4) Fluorescence Detector

For measurement of a fluorescence intensity, a detector having a flow type fluorophotometer [manufactured by Sapidyne Instrument Inc., Boise, Idaho, USA] incorporated therein was used. Fig. 1 typically shows this detector, and this detector can analyze the interaction between molecules in the liquid phase reaction by measuring the fluorescence intensity. The fluorescence intensity can be measured in units of an electrical signal by an excitation light source 3 and the detector 4 arranged with a glass cell 1 having a diameter of 1.5 mm and a length of 30 mm therebetween. The immobilized estrogen receptor 2 having the shape of beads is filled in the glass cell 1 by being banked up and laminated by a screen S consisting of 50 μm nylon mesh set in the glass cell 1. This lamination is carried out by feeding 0.7 ml of the 6.7 mg/ml immobilized estrogen receptor suspension liquid at a speed of 1.5 ml/min.

Feeding of the 17β-estradiol-bovine serum albumin-CY5 (which will be abbreviated as E2-BSA-CY5 hereinafter) only or of a mixed sample obtained from E2-BSA-CY5 and a chemical substance or a natural hormone is controlled by a suction pump, and continuously led into the glass cell at a speed of 0.5 ml/min from the direction indicated by an arrow a. At this moment, the material is measured as the fluorescence intensity by bonding to the immobilized estrogen receptor 2 in the glass cell 1. The material remaining between beads, which is not bonded to the receptor 2, is discharged from the direction indicated by an arrow b by leading only the buffer at a speed of 1.5 ml/min. Thereafter, the remaining fluorescence intensity was evaluated as a quantity of bonding. Specifically, the buffer for bonding was diluted with E2-BSA-CY5 so as to obtain approximately 400 ng/ml. It is to be noted that this concentration was appropriately adjusted in accordance with a ratio of provision of the fluorescence in E2-BSA-CY5. The same quantity of the buffer for bonding in which a natural hormone or a chemical substance with the already-known concentration was dissolved was added to the diluted solution and left to take its own course at a room temperature for 30 minutes. Incidentally, in case of a water-insoluble material, it was completely dissolved in dimethyl sulfoxide and then appropriately diluted. Further, it was left to stand and used for measurement.

B. Results

(1) Bonding of Labeled Female Sex Hormone to Immobilized Receptor

When the sample containing only E2-BSA-CY5 was continuously led into the glass cell having the immobilized estrogen receptor filled therein, increase in fluorescence intensity with time was observed. Furthermore, when the similar sample was continuously led into the glass cell having beads with no immobilized receptor for a control, increase in fluorescence intensity was also observed, but this intensity was apparently lower than that of the case using the beads having the immobilized receptor. Fig. 2 shows this result, and it was revealed that E2-BSA-CY5 was bonded to the estrogen receptor. Moreover, it was found the accumulation effect of the labeled female sex hormone can be reflected to detection since the fluorescence intensity increases with time along with a feed rate of the sample.

(2) Inhibition of Bonding of Labeled Female Sex Hormone to Immobilized Receptor by Natural Hormone

Subsequently, 17 β -estradiol and estriol which are natural female sex hormones which can be bonded to the estrogen receptor were used in the measurement system and verification was carried out. At first, the 17 β -estradiol with various concentrations was added to E2-BSA-CY5 having a fixed concentration, and the fluorescence intensity was similarly measured. As a result, as shown in Fig. 3(A), the fluorescence intensity was lowered as the concentration of the coexisting 17 β -estradiol increases. Additionally, when the 17 β -estradiol with a fixed concentration or above was contained, reduction in fluorescence intensity beyond a fixed level was not observed, and the fluorescence intensity at this moment was equal to that of the beads having no immobilized receptor, which was carried out as a contrast (see the lowermost curve in Fig. 3(A)). Incidentally, in Fig. 3(A), quantities of the added 17 β -estradiol are 0 pM, 0.05 pM, 0.5 pM, 5 pM, 50 pM and 500 pM from the upper graph curve and results when both the receptor and the 17 β estradiol were not added are shown. Therefore, it was confirmed that the 17 β -estradiol which can be bonded to the receptor competitively inhibits bonding of E2-BSA-CY5 to the receptor.

Thus, the similar examination was carried out on estriol. As shown in Fig. 3(B), it was confirmed that estriol also competitively inhibits bonding of E2-BSA-CY5 to the receptor (in the drawing, quantities of added estriol are 0 pM, 0.5 pM, 5pM, 50 pM and 500 pM from the upper graph curve, and results when both the receptor and estriol were not added are shown). On the contrary, in case of testosterone which is a male sex hormone, as shown in Fig. 3(C), it did not inhibit bonding of E2-BSA-CY5 to the receptor at concentrations for the measurement (in the drawing, quantities of added testosterone are 0 pM, 50 pM, 500 pM, 5000 pM and 500000 pM from the upper graph curve, and both the receptor and testosterone were not added).

Fig. 4 shows the relationship between the above-described natural hormone addition concentration and a ratio of reduction in fluorescence intensity. Here, it is assumed that the reduction ratio of the fluorescence intensity is 100% when no natural hormone is added, and it is expressed as a percentage that bonding of E2-BSA-CY5 to the receptor is inhibited by addition of the hormone (bonding inhibition ratio, unit: %). As a result, in case of the 17β-estradiol, reduction in fluorescence intensity can be confirmed in a range of concentration of $10^{-13}$ to $10^{-9}$ M. Further, in case of estriol, the same can be confirmed in a range of concentration of $10^{-12}$ to $10^{-9}$ M. Therefore, in the above-described measurement system, the 17 β -estradiol and estriol can be detected in the range of concentration of $10^{-13}$ to $10^{-9}$ and that of $10^{-12}$ to $10^{-9}$, respectively.

(3) Inhibition of Bonding of Labeled Female Sex Hormone to Immobilized Receptor by Chemical Substance

The examination which is the same as above was carried out with respect to chemical substances. As chemical substances, there were selected tamoxifen, bisphenol A, diethylstilbestrol (DES) and nonylphenol which are suspicious to have the extrinsic endocrine disrupting action. These substances with various concentrations were added to E2-BSA-CY5 having a fixed concentration, and reduction in fluorescence intensity was measured. As a result, the fluorescence intensity was lowered as the concentration of a coexisting chemical material is increased in all cases [Fig. 5: coexisting chemical substances are (A) tamoxifen, (B) bisphenol A, (C) DES and (D) nonylphenol, and respective added quantities are: zero, 0.5 pM, 5 pM, 50 pM, 500 pM, 5000 pM and 50000 pM and no addition of the receptor or the tamoxifen in case of (A); zero, 5 pM, 50 pM, 500 pM, 5000 pM, 50000 pM and 500000 pM and no addition of the receptor or bisphenol A in case of (B); zero, 0.05 pM, 0.5 pM, 5 pM, 500 pM and 5000 pM and no addition of the receptor or DES in case of (C); zero, 0.5 pM, 5 pM, 50 pM, 5000 pM and 50000 pM and no addition of the receptor or nonylphenol in case of (D), from the upper graph curve]. Further, when a chemical substance with a fixed or a higher concentration was added, reduction in fluorescence intensity beyond a fixed level was not observed. The fluorescence intensity at this moment was equal to that of the beads having no immobilized receptor, which was carried out as a contrast.

Based on this result, it was confirmed that these chemical substances competitively inhibit bonding of E2-BSA-CY5 to the receptor. Furthermore, assuming the reduction ratio of the fluorescence intensity is a ratio of inhibition of bonding of E2-BSA-CY5 to the receptor, Fig. 6 shows the relationship between the concentration of each added chemical substance and this ratio.

Consequently, by measuring inhibition of bonding of E2-BSA-CY5 to the receptor as a change in fluorescence intensity, tamoxifen, DES, nonylphenol and bisphenol A can be detected in concentration ranges of $10^{-12}$ to $10^{-9}$ M, $10^{-12}$ to $10^{-9}$, $10^{-9}$ to $10^{-5}$ M and $10^{-8}$ to $10^{-6}$ M, respectively. In this manner, chemical substances having the associativity with respect to the female hormone receptor can be conveniently detected at a high sensitivity in accordance with the detection method of the present invention.

Moreover, the similar operation was additionally carried out to estradiol-3-sulfate, estradiol-17-glucuronide, estradiol-3-glucuronide, estriol-3-sulfate, estriol-17-glucuronide and estriol-3-glucuronide, and all of these substances can be detected at a high sensitivity as similar to the above.

(4) Comparison with Conventional Method

As conventional methods for detecting extrinsic endocrine disrupters, there are known a radioreceptor assay, an immunoassay, a fluorescence depolarization, a method using a recombinant yeast, a method utilizing as an index bonding between a gene and a complex with a receptor by using the radioactivity, and others as mentioned above. The following table 1 shows all detection ranges when estradiol is determined as a detected substance according to these methods and the method of the present invention. Based on this result, the detection range for a chemical substance (estradiol) according to the conventional methods is 50 to 10000 pM whereas it is 1 to 100 pM in the method according to the present invention, and the effectivity of the present invention in the extrinsic endocrine disrupter detection sensitivity is obvious.

Table 1

| Method | Detection |
|---|---|
| range (pM) | |
| Radioreceptor assay | 50 to 1000 |
| Immunoassay | 370 to 37000 |
| Fluorescence depolarization | 1000 to 100000 |
| Method using recombinant yeast | 100 |
| Method according to the present invention | 1 to 100 |

Experiment II

A. Materials and Method

(1) Hormone, Anti-hormone Antibody

As hormones to be immobilized and added in a sample, 17 $\beta$-estradiol (manufactured by Wako Pure Chemical Industries, Ltd.) and estriol (manufactured by Wako Pure Chemical Industries, Ltd.) were used. Moreover, as anti-estradiol antibodies, those manufactured by Bio Design, Bio Specific, Fitzard and Coatex Bio Chemistry were used. In addition, as an anti-estriol antibody, one manufactured by Bio Stride was used. Additionally, in case of using a secondary antibody, a specific antibody against the anti-female hormone antibody (manufactured by Jackson Immuno Research Laboratories, Inc., Pennsylvania, USA) was prepared.

(2) Preparation of Hormone-immobilized Carrier

Immobilization of estradiol and estriol to a carrier (beads) was carried out by a natural adsorption method. Specifically, bovine serum albumin (manufactured by Sigma, USA) to which estradiol or estriol had been bonded was used. 100 microgram of estriol bonded bovine serum albumin was dissolved in 1 ml of physiological saline, and 200 milligram of particles of polymethyl methacrylic acid having an average particle size of 95 $\mu$m (manufactured by Ganz Chemical Co., Ltd) was added in the solution. Thereafter, concussion was performed for an hour to facilitate natural adsorption. After natural adsorption, 100 microliter of bovine serum albumin (100 milligram/ml) was added and subjected to concussion. At last, the carrier was washed with physiological saline, thereby obtaining the hormone-immobilized carrier.

(3) Preparation of Fluorescent-labeled Antibody

Fluorescent labeling of the anti-estradiol antibody or anti-estriol antibody was conducted by using a CY5 labeling kit (manufactured by Amersham Life Science, Arlington Heights, Illinois, USA). In addition, a fluorescent-labeled secondary antibody against these anti-female sex hormone antibodies was purchased from Jackson Immuno Research Laboratories, Inc. mentioned above.

(4) Fluorescence Detector

In case of using the fluorescent-labeled anti-hormone antibody, the fluorescence intensity was measured by using a detector in which a flow type fluorophotometer (manufactured by Sapidyne Instrument Inc., Boise, Idaho, USA) is incorporated with the substantially similar procedure to the experiment I.

On the other hand, Fig. 7 shows the outline of the detection method when the non-labeled anti-hormone antibody (primary antibody) is used and an antibody which is specifically bonded to the anti-hormone antibody is fluorescent-labeled to be used as a secondary antibody. As shown in Fig. 7(A), as a detector, a detector having a flow type fluorophotometer (manufactured by Sapidyne Instrument Inc., Boise, Idaho, USA) incorporated therein was used as similar to the experiment I, and an excitation light ray from an excitation light source (not shown) is caused to be incident upon female sex hormone-immobilized beads (antigen-immobilized beads) 12 filled in a glass cell 11 by a focusing optical lens 16 arranged in front of the glass cell 11, and the fluorescence emitted by excitation by the incident light is directed to the detector (not shown) by the optical lens 16. Then, the fluorescence intensity is measured by the detector in units of electrical signal. The female sex hormone-immobilized beads 12 are filled in the glass cell 11 by being banked up and laminated by a screen S set in the glass cell 11. Further, a rotary valve 17 connected to supply sources of the beads, the sample, the wash and the fluorescent-labeled secondary antibody is arranged on the upstream side of the glass cell 11. When the rotary valve operates together with a syringe pump (not shown) arranged on the downstream side, these materials are sequentially supplied into the glass cell.

As shown in Fig. 7(C), in the measurement operation, the sample previously mixed with an already-known quantity of the primary antibody is brought into contact with the antigen (female sex hormone)-immobilized beads (step 1). Here, the unreacting excessive primary antibody which is yet to react with the free female sex hormone contained in the sample reacts with the immobilized antigen on the beads and is captured. The wash is put into the cell and the sample is removed from the cell. Thereafter, a sufficient quantity of the fluorescent-labeled secondary antibody is put into the cell (step 2). In this operation, the fluorescent-labeled secondary antibody specifically reacts with the primary antibody captured by the immobilized antigen, and only a quantity corresponding to that of the bonded primary antibody is bonded to the immobilized beads. At last, the wash is fed to remove the fluorescent-labeled secondary antibody, which is yet to be bonded, from the cell (step 3), and only the labeled fluorescent material bonded through the secondary antibody to the primary antibody combined with the antigen-immobilized beads remains in the cell. A quantity of the primary antibody bonded to the antigen-immobilized beads can be detected by measuring a fluorescence intensity of that material, thereby grasping a quantity of the free antigen existing in the sample. It is to be noted that Fig. 7(B) shows a typical example of changes in fluorescence intensity detected in a series of the steps mentioned above.

B. Results

Fig. 8 shows a result of quantitative determination of estriol measured in the system having the fluorescent-labeled anti-hormone antibody, and Fig. 9 shows a result of quantitative determination of estriol and estradiol measured in the system using the fluorescent-labeled secondary antibody.

When estriol with various concentrations is added to the fluorescent-labeled antibody having a fixed concentration, a reduction ratio of the fluorescence intensity detected by the detector is determined as a ratio of inhibition of bonding to the immobilized hormone, and Fig. 7 shows the relationship between this ratio and the concentration of added free estriol at this moment. In any case, by measuring inhibition of bonding to the immobilized hormone as a change in fluorescence intensity in this manner, it is possible to achieve the detection limit which is approximately 50 to 100-fold of that of the case of similarly detecting estriol and estradiol by using the conventional enzeme-linked immunosorbent assay (ELISA). Thus, the detection method according to the present invention is proved to have the high sensitivity.

INDUSTRIAL APPLICABILITY

As described above in detail, the method and the apparatus for detecting an extrinsic endocrine disrupter according to the present invention can detect and quantitate a substance having the bonding property irrespective of types of receptors or antibodies in principle, and can detect an extrinsic endocrine disrupter at a high sensitivity which is several tens-fold of that of the conventional methods with the maximum sensitivity. Further, detection of an extrinsic endocrine disrupter according to the present invention can be carried out by measuring a fluorescence intensity, and the operation is extremely convenient.

It is said that even if an extrinsic endocrine disrupter, which is a so-called environmental hormone, exists with a very low concentration such as nanomol or picomol, it adversely affects an organism. However, the present invention can detect at a high sensitivity the environmental hormone in a sample which contains such a hormone with a very low concentration as described above, and its operation is very easy. Therefore, the present invention greatly contributes to a field of the protection of the environment as well as maintenance of ecosystem.

**Claims**

1. A method for detecting existence of an extrinsic endocrine disrupter in a sample, comprising the steps of: using an immobilized female sex hormone receptor and a fluorescent-labeled female sex hormone and carrying out a competitive bonding reaction between an already-known quantity of said fluorescent-labeled female sex hormone relative to an already-known quantity of said immobilized female sex hormone receptor and an extrinsic endocrine disrupter which would be existing in said sample; and measuring a fluorescence intensity of a fluorescent label of said fluorescent-labeled female sex hormone bonded to said immobilized female sex hormone receptor.

2. The detection method according to claim 1, wherein assuming that $F_0$ is a detection value of a fluorescence intensity detected when said fluorescent-labeled female sex hormone having a certain concentration is added into a detection container having said immobilized female sex hormone receptor filled therein and $F_1$ is a detection value of a fluorescence intensity detected when said fluorescent-labeled female sex hormone having the same concentration as the above and being mixed with said sample to be tested is added into said detection container, when said detection value $F_1$ is smaller than said detection value $F_0$, it is determined that said extrinsic endocrine disrupter exists in said sample.

3. The detection method according to claim 1, wherein assuming that $F_0$ is a detection value of a fluorescence intensity detected when said fluorescent-labeled female sex hormone having a certain concentration is added into a detection container having said immobilized female sex hormone receptor filled therein and $F_1$ is a detection value of a fluorescence intensity detected when said fluorescent-labeled female sex hormone having the same concentration as the above and being mixed with said sample to be tested is added into said detection container, a ratio of said detection value $F_1$ and said detection value $F_0$ is calculated and a quantity of said extrinsic endocrine disrupter existing in said sample is obtained by using a previously created calibration curve.

4. The detection method according to any of claims 1 to 3, wherein said female sex hormone receptor is an estrogen receptor.

5. The detection method according to any of claims 2 to 4, wherein said detection container is a cell whose cubic capacity is not more than 100 mm$^3$.

6. The detection method according to any of claims 2 to 5, wherein said detection container is of a flow type so that said sample can be continuously brought into contact with said immobilized female sex hormone receptor.

7. An apparatus for detecting an extrinsic endocrine disrupter, comprising: a detection container having an immobilized female sex hormone receptor filled therein; a light source for exciting an inside of said detection container; and a detector for detecting a fluorescence intensity in said detection container after excitation, wherein said apparatus being used in combination with a fluorescent-labeled female sex hormone.

8. The detection apparatus according to claim 7, wherein said female sex hormone receptor is an estrogen receptor.

9. The detection apparatus according to claim 7 or 8, wherein said detection container is a cell whose cubic capacity is not more than 100 mm$^3$.

10. The detection apparatus according to any of claims 7 to 9, wherein said detection container is of a flow type so that a sample can be continuously brought into contact with said immobilized female sex hormone receptor.

11. A method for detecting existence of an extrinsic endocrine disrupter in a sample, comprising the steps of: using an immobilized female sex hormone and an anti-hormone antibody relative to said female sex hormone and leading an already-known quantity of said anti-hormone antibody to said sample to be subjected to an antigen-antibody reaction with an extrinsic endocrine disrupter which would be existing in the sample; bringing a reaction liquid mixture obtained from said anti-hormone antibody and said sample into contact with an already-known quantity of said immobilized female sex hormone; and detecting a quantity of said anti-hormone antibody bonded to said immobilized female sex hormone by measuring a fluorescence intensity of a fluorescent label bonded to said anti-hormone antibody.

12. The method for detecting an extrinsic endocrine disrupter according to claim 11, wherein said anti-hormone antibody is a fluorescent-labeled anti-hormone antibody, and a quantity of said anti-hormone antibody bonded to said immobilized female sex hormone is detected by measuring a fluorescence intensity of a fluorescent label of said fluorescent-labeled anti-hormone antibody.

13. The method for detecting an extrinsic endocrine disrupter according to claim 11, wherein said anti-hormone antibody is determined as a non-labeled primary antibody, and a quantity of said anti-hormone antibody bonded to said immobilized female sex hormone is detected by using as a secondary antibody a fluorescent-labeled anti-primary-antibody antibody specifically bonded to said primary antibody, specifically bonding said secondary antibody to said primary antibody bonded to said immobilized female sex hormone and measuring a fluorescence intensity of a fluorescent label of said bonded secondary antibody.

14. The detection method according to any of claims 11 to 13, wherein assuming that $F_0$ is a detection value of a fluorescence intensity detected when said anti-hormone antibody having a certain concentration is added into a detection container having said immobilized female sex hormone filled therein and $F_1$ is a detection value of a fluorescence intensity detected when said anti-hormone antibody having the same concentration as the above and being mixed with a sample to be tested is added into said detection container, when said detection value $F_1$ is smaller than $F_0$, it is determined that an extrinsic endocrine disrupter exists in said sample.

15. The detection method according to any of claims 11 to 13, wherein assuming that $F_0$ is a detection value of a fluorescence intensity detected when said anti-hormone antibody having a certain concentration is added into a detection container having said immobilized female sex hormone filled therein, and $F_1$ is a detection value of a fluorescence intensity detected when said anti-hormone antibody having the same concentration as the above and being mixed with a sample to be tested is added into said detection container, a ratio of said detection value $F_1$ and said detection value $F_0$ is calculated, and a quantity of an extrinsic endocrine disrupter existing in said sample is obtained by using a previously created working curve.

16. The detection method according to claim 14 or 15, wherein said detection container is a cell whose cubic capacity is not more than 100 mm$^3$.

17. The detection method according to any of claims 12 to 16, wherein said detection container is of a flow type so that said sample can be continuously brought into contact with said immobilized female sex hormone.

18. An apparatus for detecting an extrinsic endocrine disrupter, comprising: a detection container having an immobilized female sex hormone filled therein; a light source for exciting an inside of said detection container; and a detector for detecting a fluorescence intensity in said detection container after excitation, wherein said apparatus being used in combination with an anti-hormone antibody relative to said fluorescent-labeled female sex hormone.

19. An apparatus for detecting an extrinsic endocrine disrupter, comprising: a detection container having an immobilized female sex hormone filled therein; a light source for exciting an inside of said detection container; and a detector for detecting a fluorescence intensity in said detection container after excitation, wherein said apparatus being used in combination with an anti-hormone primary antibody relative to said female sex hormone and a fluorescent-labeled secondary antibody specifically bonded to said anti-hormone antibody.

Fig. 1

Fig. 2

Fig. 3

Fig. 4

concentration of hormone (M)

inhibition ratio of bonding of E2-BSA-CY5 (%)

● ; 17β - estradiol 、 ▲ ; estriol 、 ■ ; testosterone

Fig. 5

Fig. 6

concentration of chemical substance (M)

inhibition ratio of bonding of E2-BSA-CY5 (%)

● ; tamoxifen 、 ▲ ; nonylphenol 、 ■ ; bisphenol A
▼ ; D E S

Fig. 7

(A)

beads
sample
wash
fluorescent-labeled
secondary antibody

17

11

12

S

add

excitation light

fluorescence

16

syringe pump

(B)

step 1    step 2    step 3

fluorescence intensity

0    100    300

analysis time (sec.)

(C)

antigen    step 1     step 2     step 3

primary antibody

fluorescent-labeled
secondary antibody

Fig. 8

concentration of estriol [picomole]

Fig. 9

concentration of estradiol [picomole]

concentration of estriol [picomole]

## INTERNATIONAL SEARCH REPORT

| | International application No. |
|---|---|
| | PCT/JP00/01813 |

A. CLASSIFICATION OF SUBJECT MATTER
Int.Cl$^7$  G01N33/53, G01N33/543, G01N33/566

According to International Patent Classification (IPC) or to both national classification and IPC

B. FIELDS SEARCHED

Minimum documentation searched (classification system followed by classification symbols)
Int.Cl$^7$  G01N33/53, G01N33/543, G01N33/566

Documentation searched other than minimum documentation to the extent that such documents are included in the fields searched
Jitsuyo Shinan Koho          1922-1996    Toroku Jitsuyo Shinan Koho  1994-2000
Kokai Jitsuyo Shinan Koho  1971-2000    Jitsuyo Shinan Toroku Koho  1996-2000

Electronic data base consulted during the international search (name of data base and, where practicable, search terms used)
WPI,BIOSIS

C. DOCUMENTS CONSIDERED TO BE RELEVANT

| Category* | Citation of document, with indication, where appropriate, of the relevant passages | Relevant to claim No. |
|---|---|---|
| X<br>Y | Environmental Health Perspectives Vol.106,No.9 (1998)<br>p.551-557,<br>page 552, column 1, lines 29-52; Materials and Methods | 1-5,11-16<br>6,7-10,17,<br>18,19 |
| Y | JP, 9-273993, A (Matsushita Electric Ind. Co., Ltd.),<br>21 October, 1997 (21.10.97),<br>Full text; Fig. 1  (Family: none) | 6,7-10,<br>17,18,19 |
| A | JP, 6-265550, A (Kyowa Medex Co., Ltd.),<br>22 September, 1994 (22.09.94)<br>& WO, 94/22012, A | 1-19 |
| A | JP, 6-43164, A (Olympus Optical Company Limited),<br>18 February, 1994 (18.02.94)    (Family: none) | 1-19 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>19 June, 2000 (19.06.00) | Date of mailing of the international search report<br>27 June, 2000 (27.06.00) |
|---|---|
| Name and mailing address of the ISA/<br>Japanese Patent Office | Authorized officer |
| Facsimile No. | Telephone No. |

Form PCT/ISA/210 (second sheet) (July 1992)